**Europäisches Patentamt**

**European Patent Office**

**Office européen des brevets**

(11) Veröffentlichungsnummer : **0 155 992
B1**

(12) **EUROPÄISCHE PATENTSCHRIFT**

(45) Veröffentlichungstag der Patentschrift :
29.07.87

(21) Anmeldenummer : **84112018.1**

(22) Anmeldetag : **06.10.84**

(51) Int. Cl.⁴ : **C 07 C 15/00**, C 07 C 7/08//
C10G21/20

(54) Verfahren zur Abtrennung von Aromaten aus Kohlenwasserstoffgemischen beliebigen Aromatengehaltes.

(30) Priorität : **14.03.84 DE 3409307**

(43) Veröffentlichungstag der Anmeldung :
**02.10.85 Patentblatt 85/40**

(45) Bekanntmachung des Hinweises auf die Patenterteilung : **29.07.87 Patentblatt 87/31**

(84) Benannte Vertragsstaaten :
**AT BE DE FR GB IT NL**

(56) Entgegenhaltungen :
**DE-A- 1 568 940**

(73) Patentinhaber : **Krupp Koppers GmbH
Altendorfer Strasse 120
D-4300 Essen 1 (DE)**

(72) Erfinder : **Berns, Horst
Lindenhof 31
D-4330 Mülheim/Ruhr (DE)**

Jouve, 18, rue St-Denis, 75001 Paris, France

## Beschreibung

Die Erfindung betrifft ein Verfahren zur Abtrennung von Aromaten aus Kohlenwasserstoffgemischen beliebigen Aromatengehaltes, die als nichtaromatische Bestandteile insbesondere Paraffine und Cycloparaffine enthalten, durch Extraktivdestillation, bei der N-substituierte Morpholine, deren Substituenten nicht mehr als sieben C-Atome aufweisen, als selektives Lösungsmittel verwendet werden.

Ein Aromatengewinnungsverfahren der vorstehend genannten Art ist bereits in der DE-PS 1 568 940 und verschiedenen anderen Publikationen beschrieben worden. Dieses Verfahren wurde in der Zwischenzeit auf verschiedenen großtechnischen Anlagen eingesetzt und hat sich dort in der Praxis auch bewährt. Es hat sich dabei allerdings gezeigt, daß bei der Aufarbeitung bestimmter Einsatzprodukte, wie beispielsweise einer aus Pyrolysebenzin gewonnenen Rohbenzolfraktion, trotz Einhaltung analoger Verfahrensbedingungen von Fall zu Fall gewisse Schwankungen in der Aromatenausbeute sowie in der Reinheit der gewonnenen Produkte auftraten, für die es zunächst keine plausible Erklärung gab. Obwohl die aufgetretenen Schwankungen von der Größenordnung her verhältnismäßig gering waren, so stellen sie doch im großtechnischen Betrieb eine unerwünschte Beeinträchtigung dar, weil dadurch nicht nur die Reinheit der gewonnenen Produkte verändert wird, sondern gleichzeitig auch die Aromatenausbeute und/oder der Energieverbrauch der Anlage beeinflußt wird. So wurden beispielsweise bei der Aufarbeitung einer aus Pyrolysebenzin gewonnenen Rohbenzolfraktion sowohl Schwankungen des Nichtaromatengehaltes im gewonnenen Reinbenzol als auch des Benzolgehaltes in der Nichtaromatenfraktion beobachtet. Gleichzeitig traten auf dem sogenannten Regelboden der Extraktivdestillationskolonne Temperaturschwankungen auf.

Der Erfindung liegt deshalb die Aufgabe zu Grunde, das Verfahren der eingangs genannten Art dahingehend zu verbessern, daß derartige Schwankungen möglichst weitgehend vermieden werden und gleichzeitig das Verfahren in Bezug auf die Reinheit der gewonnenen Aromaten- und Nichtaromatenfraktion sowie die Ausbeute optimiert wird.

Das der Lösung dieser Aufgabe dienende Verfahren der eingangs genannten Art ist erfindungsgemäß dadurch gekennzeichnet, daß in dem als Einsatzprodukt dienenden Kohlenwasserstoffgemisch ein dessen Gehalt an schweren Nichtaromaten, deren Siedepunkte wenigstens 14 °C über dem Siedepunkt der zu gewinnenden reinen aromatischen Verbindung liegen, proportionaler Gehalt an leichten Nichtaromaten, deren Siedepunkte wenigstens 38 °C unter dem Siedepunkt der zu gewinnenden reinen aromatischen Verbindung liegen, eingestellt wird, wobei das Gewichtsverhältnis von schweren Nichtaromaten zu Leichten Nichtaromaten höchstens 1 : 0,4 betragen soll und die zur Einstellung dieses Gewichtsverhältnisses erforderlichen leichten Nichtaromaten flüssig oder dampfförmig in das Unterteil der Extraktivdestillationskolonne unterhalb der Einsatzproduktaufgabe eingeleitet oder dem Einsatzprodukt vor dessen Eintritt in die Extraktivdestillationskolonne zugesetzt werden.

Die Erfindung geht dabei von der Erkenntnis aus, daß zwischen dem Gehalt an schweren Nichtaromaten im Einsatzprodukt und dessen Gehalt an leichten Nichtaromaten eine Beziehung besteht, durch die die Reinheit der gewonnenen Produkte sowie die Fahrweise der Anlage beeinflußt wird. Welche Verbindungen hierbei als schwere und als leichte Nichtaromaten anzusehen sind, ergibt sich hierbei aus der Lage ihrer Siedepunkte und ist außerdem, wie sich aus den weiter oben aufgeführten erfindungsgemäßen Definitionen ergibt, abhängig vom Siedepunkt der jeweils zu gewinnenden reinen aromatischen Verbindung. Das heißt, wenn durch das erfindungsgemäße Verfahren beispielsweise ausschließlich Reinbenzol (KP 80 °C) gewonnen werden soll, so sind im Sinne der weiter oben aufgeführten Definition als schwere Nichtaromaten alle nichtaromatischen Verbindungen anzusehen, deren Siedepunkt wenigstens 94 °C beträgt, während als leichte Nichtaromaten alle nichtaromatischen Verbindungen anzusehen sind, deren Siedepunkt nicht mehr als 42 °C beträgt. Bei der Gewinnung von Toluol oder Xylol verschieben sich diese Siedepunktgrenzen natürlich entsprechend der Lage der Siedepunkte von Toluol und Xylol. In der vorliegenden Verfahrensbeschreibung wird jedoch der Einfachheit halber stets nur der Sammelbegriff schwere bzw. Leichte Nichtaromaten verwendet.

Es hat sich auf Grund eingehender Untersuchungen gezeigt, daß sich die schweren Nichtaromaten aus der bei der Extraktivdestillation als Extrakt im Sumpf der Extraktivdestillationskolonne anfallenden Aromatenfraktion dann weitgehend abtreiben lassen, wenn im Einsatzprodukt die leichten Nichtaromaten in ausreichendem Umfang vorhanden sind. Hierbei handelt es sich in erster Linie um Paraffine mit einem C-Atom weniger, als in der zu gewinnenden aromatischen Verbindung vorhanden ist. Diese leichten Nichtaromaten wirken quasi als Strippdampf und sorgen so für den Abtrieb der schweren Nichtaromaten aus der im Sumpf der Extraktivdestillationskolonne befindlichen Aromatenfraktion sowie dafür, daß das Nichtaromaten-Gemisch im Kolonnenoberteil einen niedrigen Taupunkt hat. Um dies sicher zu gewährleisten, ist es notwendig, daß das weiter oben genannte, erfindungsgemäß beanspruchte Gewichtsverhältnis von schweren zu leichten Nichtaromaten im Einsatzprodukt eingehalten wird.

Es ist erfindungsgemäß erforderlich, durch einen entsprechenden Zusatz von Leichten Nichtaromaten das Gewichtsverhältnis nach Anspruch 1 einzustellen. Die hierfür erforderlichen leichten Nichtaromaten können dabei flüssig oder dampfförmig in das Unterteil der Extraktivdestillationskolonne unterhalb der Einsatzproduktaufgabe eingeleitet oder dem Einsatzprodukt vor dessen Eintritt in die Extraktivdestillationskolonne zugesetzt werden. Sofern im Einsatzprodukt mehr leichte Nichtaromaten als erforderlich vorhanden sind, ist das im Prinzip zwar unschädlich. Es muß aber berücksichtigt werden, daß die leichten Nichtaromaten bei der Extraktivdestillation über Kopf aus der Extraktivdestillationskolonne abgetrieben werden müssen und die physikalischen Eigenschaften der übrigen Nichtaromaten beeinflussen, z. B. erhöhen sie den Dampfdruck. Es ist deshalb vorteilhaft, wenn der Zusatz an leichten Nichtaromaten zum Einsatzprodukt so bemessen wird, daß im Einsatzprodukt ein Verhältnis von schweren zu leichten Nichtaromaten von 1 : 5,0 nicht unterschritten wird.

Die leichten Nichtaromaten, welche zur Einstellung des notwendigen Gewichtsverhältnisses von schweren Nichtaromaten zu leichten Nichtaromaten in den Unterteil der Extraktivdestillationskolonne eingeleitet oder dem Einsatzprodukt vor dessen Eintritt in diese Kolonne zugesetzt werden müssen, können ganz oder teilweise aus den Kopfproduktdämpfen der Extraktivdestillationskolonne durch entsprechende partielle Kondensation gewonnen und zur Aufgabestelle zurückgeführt werden.

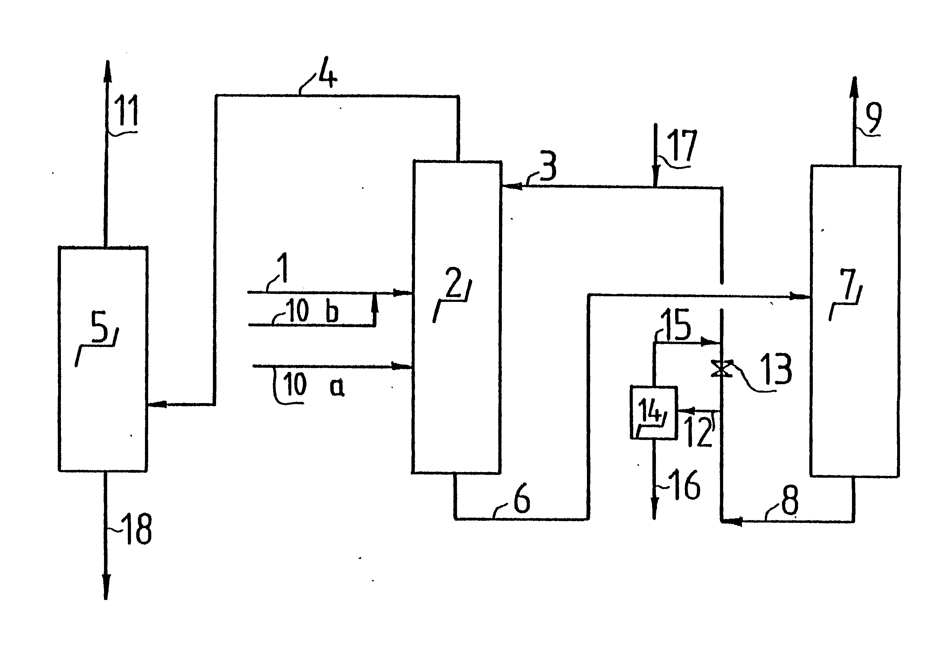

Das in der Abbildung dargestellte Fließschema gibt den Verfahrensgang der erfindungsgemäßen Extraktivdestillation in vereinfachter Form wieder.

Hierbei handelt es sich um das bei Extraktivdestillationen übliche Verfahrensschema. Das aufzutrennende Kohlenwasserstoffgemisch, das als Einsatzprodukt dient, wird durch die Leitung 1 in den mittleren Teil der mit Böden oder ähnlichen Einbauten versehenen Extraktivdestillationskolonne 2 eingeleitet. Das Einsatzprodukt ist dabei entweder vor dem Eintritt in die Extraktivdestillationskolonne 2 auf Temperaturen dicht unterhalb des Siedepunkte erhitzt worden, oder es wird bereits im teilverdampften Zustand in die Extraktivdestillationskolonne 2 eingeführt. Durch die Leitung 3 wird das selektive Lösungsmittel am Kopf in die Extraktivdestillationskolonne 2 eingeleitet und fließt über die Einbauten dieser Kolonne herab nach unten, wobei es die dampfförmigen Aromaten aufnimmt. Die nichtaromatischen Kohlenwasserstoffe entweichen durch Leitung 4 am Kopf der Kolonne und können in der Kondensationseinrichtung 5 partiell kondensiert werden. Das flüssige Sumpfprodukt der Extraktivdestillationskolonne (Extrakt) besteht aus dem selektiven Lösungsmittel und den darin gelösten Aromaten und wird durch die Leitung 6 aus der Extraktivdestillationskolonne 2 abgezogen und gelangt in die Kolonne 7, in der die Aromaten destillativ vom selektiven Lösungsmittel abgetrennt werden. Das selektive Lösungsmittel wird durch die Leitung 8 aus dem Kolonnensumpf entfernt und gelangt über die Leitung 3 wieder in die Extraktivdestillationskolonne 2 zurück, während die Aromatendämpfe über die Leitung 9 über Kopf aus der Kolonne 7 entweichen und in nicht dargestellten Kondensationseinrichtungen kondensiert werden.

Der Zusatz der erforderlichen leichten Nichtaromaten erfolgt über die Leitung 10 a oder 10 b. Hierbei können durch partielle Kondensation gewonnene leichte Nichtaromaten aus der Leitung 11 zur Aufgabestelle zurückgeführt werden. Da sich im Laufe der Zeit im selektiven Lösungsmittel Verunreinigungen anreichern können, ist im Bereich der Leitung 8 die Abzweigleitung 12 vorgesehen, durch die bei entsprechender Stellung des Ventils 13 eine Teilmenge des selektiven Lösungsmittels zur Regenerierungseinrichtung 14 gelangen kann. Das regenerierte lösungsmittel wird durch die Leitung 15 wieder in den Kreislauf (Leitung 3) zurückgeführt, während die ausgeschiedenen Verunreinigungen durch die Leitung 16 aus der Regeneriereinrichtung 14 abgezogen werden. Die Leitung 17 dient der Zufuhr von frischem Lösungsmittel.

Über die Leitung 18 werden die Nichtaromaten aus der Kondensationseinrichtung 5 ausgeschleust bzw. einer Nachbehandlung zugeführt, die nicht über die Leitung 11 abgezogen und zur Extraktivdestillationskolonne 2 zurückgeführt werden.

Selbstverständlich sind bei der praktischen Anwendung des erfindungsgemäßen Verfahrens gewisse Abweichungen von dem vorstehend skizzierten Verfahrensgang der Extraktivdestillation möglich. Diese Abweichungen stehen jedoch in keinem Zusammenhang mit der vorliegenden Erfindung und brauchen deshalb nicht näher erläutert zu werden.

Abschließend soll die Wirkung des erfindungsgemäßen Verfahrens durch die nachfolgenden Vergleichsversuche belegt werden. Hierbei wurde als Einsatzprodukt eine aus Pyrolysebenzin gewonnene Rohbenzolfraktion verwendet, die folgende Zusammensetzung aufweis :

(Siehe Tabelle Seite 4 f.)

|  | Kp | Gew.-% |
|---|---|---|
| Σi-Pentane | 9,5-28,9 °C | 0,12 |
| n-Pentan | 36,1 °C | 0,08 |
| | Leichte Nichtaromaten = | 0,20 |
| n-Heptan | 98,4 °C | 0,52 |
| 1 cis 2 Dimethylcyclopentan | 99,5 °C | 0,03 |
| Methylcyclohexan | 100,9 °C | 0,13 |
| Äthylcyclopentan | 103,4 °C | 0,03 |
| | Schwere Nichtaromaten = | 0,71 |
| Benzol | 80,1 °C | 74,18 |
| | Σ = | 75,09 |

Die restlichen 24,91 Gew.-% sind zwischen den leichten und schweren Nichtaromaten siedende Nichtaromaten.

Mit diesem Einsatzprodukt, aus dem das Reinbenzol durch Extraktivdestillation mit N-Formylmorpholin als selektivem Lösungsmittel abgetrennt wurde, wurden unter Einhaltung analoger Verfahrensbedingungen vier Vergleichsversuche durchgeführt. Hierbei wurde bei Versuch 1 ohne einen weiteren Zusatz von leichten Nichtaromaten gearbeitet, während hierfür bei den Versuchen 2 bis 4 jeweils ein steigender Zusatz von $C_5$-Nichtaromaten (Pentane) erfolgt. Die Ergebnisse sind in der nachfolgenden Tabelle zusammengefaßt :

| Versuchs-Nr. | 1 | 2 | 3 | 4 |
|---|---|---|---|---|
| $C_5$-Zusatz in 100 Teilen Einsatzprodukt zur ED-Kolonne | 0,00 | 0,25 | 1,50 | 1,70 |
| $\dfrac{\text{Schwere Nichtaromaten im Einsatz}}{\text{Leichte Nichtaromaten im Einsatz}}$ | 1 : 0,28 | 1 : 0,63 | 1 : 2,43 | 1 : 2,72 |
| t Heizdampf zur ED-Kolonne/t Reinbenzol | 0,191 | 0,190 | 0,191 | 0,191 |
| Benzolgehalt der Nichtaromaten | 9,60 % | 8,10 % | 6,10 % | 4,80 % |
| $C_5$-Gehalt der Nichtaromaten | 0,70 % | 1,59 % | 5,93 % | 6,68 % |
| Nichtaromatengehalt des Reinbenzols | 0,108 % | 0,098 % | 0,074 % | 0,060 % |
| $\dfrac{\text{Reinbenzolprodukt 100}}{C_6H_6 \text{ im Einsatz}}$ (Ausbeute) | 96,41 % | 97,00 % | 97,68 % | 98,19 % |

Ein Vergleich der Versuchsergebnisse zeigt, daß mit steigendem Zusatz von leichten Nichtaromaten zum Einsatzprodukt sowohl die Reinheit der gewonnenen Fraktionen (Aromaten und Nichtaromaten) als auch die Benzolausbeute verbessert wurde, ohne daß es hierbei zu einer Erhöhung des Heizdampfbedarfs in der Extraktivdestillationskolonne kam. Damit dürfte die vorteilhafte Wirkung des erfindungsgemäßen Verfahrens eindeutig unter Beweis gestellt worden sein.

**Patentansprüche**

1. Verfahren zur Abtrennung von Aromaten aus Kohlenwasserstoffgemischen beliebigen Aromatengehaltes, die als nichtaromatische Bestandteile insbesondere Paraffine und Cycloparaffine enthalten, durch Extraktivdestillation, bei der N-substituierte Morpholine, deren Substituenten nicht mehr als sieben C-Atome aufweisen, als selektives Lösungsmittel verwendet werden, dadurch gekennzeichnet, daß in dem als Einsatzprodukt dienenden Kohlenwasserstoffgemisch ein dessen Gehalt an schweren Nichtaromaten, deren Siedepunkte wenigstens 14 °C über dem Siedepunkt der zu gewinnenden reinen aromatischen Verbindung liegen, proportionaler Gehalt an leichten Nichtaromaten, deren Siedepunkte wenigstens 38 °C unter dem Siedepunkt der zu gewinnenden reinen aromatischen Verbindung liegen, eingestellt wird, wobei das Gewichtsverhältnis von schweren Nichtaromaten zu leichten Nichtaromaten Löchstens 1 : 0,4 betragen soll und die zur Einstellung dieser Gewichtsverhältnisse erforderlichen leichten Nichtaromaten flüssig oder dampfförmig in das Unterteil der Extraktivdestillationskolonne unterhalb der Einsatzproduktaufgabe eingeleitet oder dem Einsatzprodukt vor dessen Eintritt in die Extraktivdestillationskolonne zuge setzt werden.

2. Verfahren nach Anspruch 1, dadurch gekennzeichnet, daß die Zugabe von leichten Nichtaromaten so bemessen wird, daß im Einsatzprodukt ein Gewichtsverhälnis von schweren Nichtaromaten zu leichten Nichtaromaten von 1 : 5,0 nicht unterschritten wird.

3. Verfahren nach den Ansprüchen 1 und 2, dadurch gekennzeichnet, daß die zuzusetzenden Leichten Nichtaromaten ganz oder teilweise aus den Kopfproduktdämpfen der Extraktivdestillationskolonne zurückgewonnen werden.

## Claims

1. Process for separating aromatics from hydrocarbon mixtures of any aromatics content, which contain especially paraffins and cycloparaffins as the non-aromatic constituents, by extractive distillation in which N-substituted morpholines, the substituents of which do not have more than seven C atoms, are used as the selective solvent, characterised in that, in the hydrocarbon mixture used as the feed material, the content of heavy non-aromatics, having boiling points at least 14 °C above the boiling point of the pure aromatic compound to be produced, is adjusted proportional to its content of light non-aromatics, having boiling points at least 38 °C below the boiling point of the pure aromatic compound to be produced, whereat the weight ratio of heavy non-aromatics to light non-aromatics is to be at most 1 : 0.4 and for adjusting this weight ratios the required light non-aromatics are introduced in the liquid or vapour form into the lower section of the extractive distillation column below the feed point or are added to the feed material before the latter enters the extractive distillation column.

2. Process according to Claim 1, characterised in that light non-aromatics are added in such a quantity that a weight ratio of heavy non-aromatics to light non-aromatics in the feed material is not lower than 1 : 5:0.

3. Process according to Claims 1 and 2, characterised in that the light non-aromatics to be added are wholly or partially recovered from the top product vapours of the extractive distillation column.

## Revendications

1. Procédé pour séparer des fractions aromatiques de mélanges d'hydrocarbures d'une teneur quelconque en fractions aromatiques, qui présentent, comme constituants non-aromatiques, en particulier des paraffines et des cyclo-paraffines, par une distillation extractive lors de laquelle on utilise, comme solvant sélectif, des morpholines N-substituées, dont les substituants ne présentent pas plus de 7 atomes de carbone, caractérisé par le fait que l'on établit dans le mélange d'hydrocarbures, servant de produit de mise en œuvre, une teneur en fractions non-aromatiques légères dont les points d'ébullition, de 38 °C au moins, se situent au-dessous du point d'ébullition du composé aromatique pur à obtenir, ladite teneur étant proportionnelle à la teneur en fractions non-aromatiques lourdes du produit de mise en œuvre, dont les points d'ébullition sont situés au moins 14 °C au-dessus du point d'ébullition du composé aromatique pur à obtenir, tandis que le rapport pondéral des fractions non-aromatiques lourdes aux fractions non-aromatiques légères doit être au plus égal à 1 : 0,4 et que les fractions non-aromatiques légères nécessaires pour établir ce rapport pondéral sont introduites à l'état liquide ou à l'état de vapeur dans la partie inférieure de la colonne de distillation extractive, au-dessous de l'entrée du produit de mise en œuvre, ou bien qu'elles sont ajoutées à ce dernier avant son entrée dans la colonne de distillation extractive.

2. Procédé selon la revendication 1, caractérisé par le fait que l'addition des fractions non-aromatiques légères est mesurée de manière, que dans le procédé de mise en œuvre on ne dépasse pas par défaut un rapport pondéral de 1 : 5,0 entre les fractions non-aromatiques lourdes et les fractions non-aromatiques légères.

3. Procédé selon les revendications 1 et 2, caractérisé par le fait que les fractions non-aromatiques légères à ajouter sont totalement ou partiellement récupérées à partir des vapeurs du produit de tête de la colonne de distillation extractive.

0 155 992